# FASCICULE DE BREVET EUROPEEN

(11) **EP 2 612 850 B1**
(45) Date de publication et mention de la délivrance du brevet: **18.09.2013**
(21) Numéro de dépôt: 13150296.5
(22) Date de dépôt: 04.01.2013
(51) Int. Cl.: C07D 209/42, A61K 31/403

(54) **Forme cristalline delta du sel d'arginine du périndopril, son procédé de préparation, et les compositions pharmaceutiques qui la contiennent**
Krystallische Delta-Form des Arginin-Salz von Perindopril, ein Verfahren zu ihrer Herstellung und die enthaltenden pharmazeutische Zusammensetzungen
Delta crystalline form of the arginine salt of perindopril, a process for its preparation, and pharmaceutical compositions containing it

(30) Priorité: 05.01.2012 FR 1200033
(43) Date de publication de la demande: 10.07.2013
(73) Titulaire: Les Laboratoires Servier, 92284 Suresnes Cedex (FR)
(72) Inventeur: Linol, Julie, 76770 Malaunay (FR); Laurent, Stéphane, 76190 Valliquerville (FR); Grenier, Arnaud, 76110 Breaute (FR); Mathieu, Sébastien, 76290 Montivillers (FR)

(56) Documents cités:
- EP-A1- 1 354 873
- WO-A1-2007/099217
- WO-A2-2007/099216
- WO-A2-2009/157018
- SI-A- 23 001
- TELEJKO, ELWIRA: "Perindopril arginine : benefits of a new", CURRENT MEDICAL RESEARCH AND OPINION, vol. 23, no. 5, 2007, pages 953-960, XP008152666,

## Description

La présente invention concerne la forme cristalline delta du sel de L-arginine du périndopril de formule (I) : 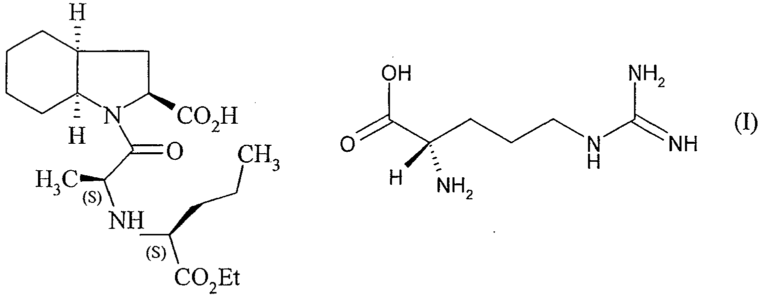 son procédé de préparation ainsi que les compositions pharmaceutiques qui la contiennent.

Le périndopril, ainsi que ses sels pharmaceutiquement acceptables, et plus particulièrement son sel d'arginine, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.

Ces deux actions contribuent aux effets bénéfiques du périndopril dans les maladies cardiovasculaires, tout particulièrement l'hypertension artérielle, l'insuffisance cardiaque et la maladie coronaire stable.

Le périndopril, sa préparation et son utilisation en thérapeutique ont été décrits dans le brevet européen EP 0 049 658.

Le sel d'arginine du périndopril a été décrit pour la première fois dans le brevet européen EP 1 354 873.

Les formes cristallines alpha et bêta du sel d'arginine du périndopril ont été décrites dans les brevets européens EP 1 989 182 et EP 2 016 051.

La forme cristalline gamma du sel d'arginine du périndopril a été décrite dans la demande de brevet WO 2009/157018.

Un procédé d'obtention du périndopril arginine a été décrit dans le brevet SI 23001.

Compte tenu de l'intérêt pharmaceutique du périndorpil arginine, il était primordial de l'obtenir avec une excellente stabilité.

Plus spécifiquement, la présente invention concerne la forme cristalline delta du composé de formule (I).

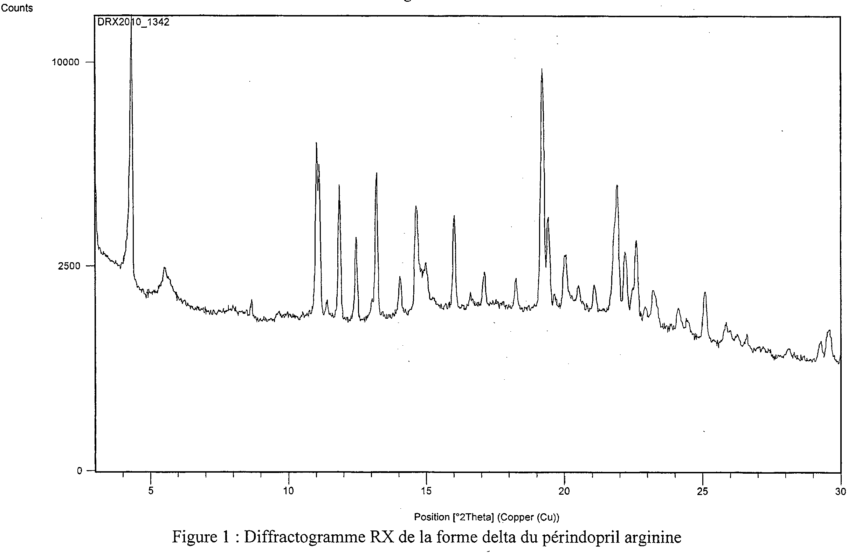

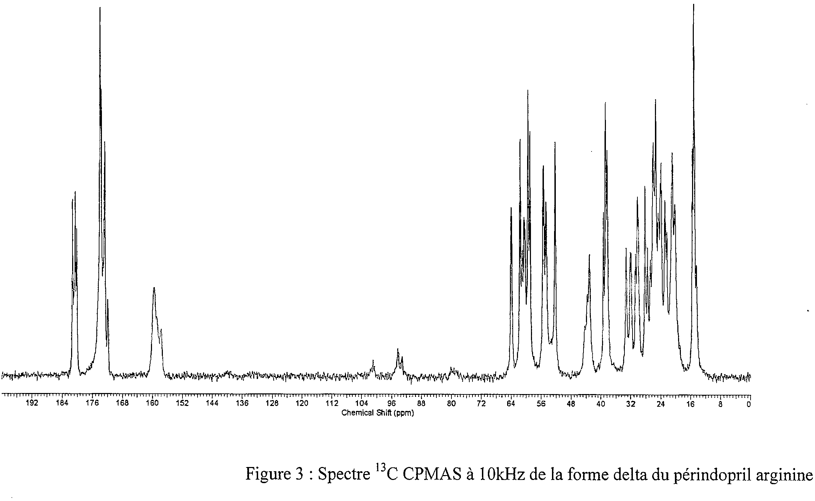

La forme cristalline delta du périndopril arginine selon l'invention peut être caractérisée par son diffractogramme RX selon la figure 1 et/ou par son spectre de RMN du solide selon la figure 3.

En l'absence d'excipients et d'impuretés, la forme cristalline delta du périndopril arginine selon l'invention peut être caractérisée par le diagramme de diffraction X sur poudre suivant, mesuré sur un diffractomètre à anticathode de cuivre et exprimé en termes de distance inter-réticulaire d, d'angle de Bragg 2 thêta et d'intensité relative, exprimée en pourcentage par rapport à la raie la plus intense :

| **Angle 2 thêta (°)** | **Distance inter-réticulaire d [Å]** | **Intensité relative [%]** |
|---|---|---|
| 4,34 | 20,37 | 66,2 |
| 5,57 | 15,86 | 5,2 |
| 11,04 | 8,02 | 57,5 |
| 11,15 | 7,94 | 47,5 |
| 11,87 | 7,454 | 35,0 |
| 12,47 | 7,09 | 17,9 |
| 13,21 | 6,70 | 33,6 |
| 14,06 | 6,30 | 6,6 |
| 14,64 | 6,05 | 31,8 |
| 16,03 | 5,53 | 17,5 |
| 17,11 | 5,18 | 5,6 |
| 18,27 | 4,85 | 4,1 |
| 19,23 | 4,61 | 100 |
| 19,44 | 4,57 | 17,8 |
| 20,04 | 4,43 | 13,6 |
| 21,11 | 4,21 | 3,7 |
| 21,93 | 4,05 | 23,0 |
| 22,20 | 4,00 | 16,9 |
| 22,61 | 3,93 | 21,2 |
| 23,21 | 3,83 | 4,5 |
| 24,30 | 3,66 | 2,3 |
| 25,09 | 3,55 | 9,4 |
| 25,95 | 3,43 | 1,7 |
| 29,54 | 3,02 | 4,2 |

Chaque raie est considérée avoir une précision de ± 0,2° en 2-thêta.

Les intensités relatives sont données à titre indicatif.

Le spectre de diffraction X sur poudre a été mesuré avec les conditions expérimentales suivantes :
- Diffractomètre Panalytical X'Pert Pro
- Détecteur X'Celerator
- Anticathode cuivre, voltage 40KV, intensité 30mA ;
- Montage en transmission; échantillon fixe ;
- Température : ambiante ;
- Domaine de mesures : 3° à 40° ;
- Incrémentation entre chaque mesure : 0,017° ;
- Temps de mesure par pas : 49s ;
- Pas de référence interne ;
- Données expérimentales traitées avec le logiciel X'Pert Highscore (Version 2.2a)

En présence d'impuretés ou d'excipients, notamment en présence de lactose, certains pics de diffraction RX de la forme delta du périndopril arginine selon l'invention peuvent être masqués.

Selon la nature des excipients ou impuretés, la forme cristalline delta du périndopril arginine selon l'invention pourra alors être caractérisée par les pics de diffraction RX sur poudre suivants, mesurés sur un diffractomètre à anticathode de cuivre et exprimés en termes d'angle 2-thêta (°) : 4,3, 11,0, 11,1, 13,2, 14,6, 16,0 et 21,9 ; ou 4,3, 11,0, 11,1, 11,9, 13,2, 14,6, 19,2, 21,9 et 22,6 ; ou 4,3, 11,0, 11,1, 11,9, 12,5, 13,2, 14,6, 16,0, 19,2, 19,4, 21,9, 22,2 et 22,6.

La forme cristalline delta du sel d'arginine du périndopril a également été caractérisée par spectroscopie RMN à l'état solide.

Le spectre ¹³C RMN à l'état solide a été enregistré à température ambiante à l'aide d'un spectromètre Bruker SB Avance avec une sonde de type 4 mm CP/MAS SB VTN dans les conditions suivantes :
- Fréquence : 125,76 MHz,
- Largeur spectrale : 40 kHz,
- Vitesse de rotation de l'échantillon à l'angle magique (Magic Angle Spinning Rate) : 10 kHz,
- Séquence d'impulsion CP (Cross Polarization) avec découplage SPINAL64 (puissance de découplage de 80 kHz),
- Délai de répétitions : 10 s,
- Temps d'acquisition : 47 ms,
- Temps de contact : 4 ms
- Nombre de scans : 4096.

Un élargissement des raies de 5 Hz a été appliqué avant transformation de Fourier.

Le spectre ainsi obtenu a été référencé par rapport à un échantillon d'adamantane (pic haute fréquence de l'adamantane fixé à 38,48 ppm).

Les pics observés ont été rassemblés dans le tableau suivant (exprimés en ppm ± 0,2 ppm) :

| Pic n° | Déplacement chimique (ppm) | Pic n° | Déplacement chimique (ppm) |
|---|---|---|---|
| 1 | 181,2 | 10 | 38,4 |
| 2 | 180,5 | 11 | 15,6 |
| 3 | 180,1 | 12 | 15,2 |
| 4 | 174,0 | 13 | 15,0 |
| 5 | 173,7 | 14 | 14,5 |
| 6 | 172,7 | | |
| 7 | 172,0 | | |
| 8 | 39,3 | | |
| 9 | 38,8 | | |

L'invention s'étend aussi à un procédé de préparation de la forme cristalline delta du sel d'arginine du périndopril par cristallisation ou recristallisation du sel d'arginine du périndopril dans un mélange binaire d'acétonitrile, d'acétate d'éthyle ou de méthyl tert-butyl éther et de diméthylsulfoxyde ou un mélange ternaire d'acétonitrile, de diméthylsulfoxyde et de toluène, à une température supérieure à 20°C.

Dans le cas d'un procédé par cristallisation, le sel d'arginine du périndopril peut être obtenu à partir d'un autre sel de périndopril, par exemple le sel de tert-butylamine, que l'on fait réagir avec un acide pour obtenir le périndopril sous forme d'acide libre, que l'on salifie par l'arginine dans un mélange binaire d'acétonitrile, d'acétate d'éthyle ou de méthyl tert-butyl éther et de diméthylsulfoxyde ou un mélange ternaire d'acétonitrile, de diméthylsulfoxyde et de toluène.

Dans le cas d'un procédé par recristallisation, le sel d'arginine du périndopril utilisé comme matière première peut être sous forme anhydre ou hydratée, sous forme amorphe ou sous n'importe quelle forme cristalline.

Lorsque qu'un mélange binaire d'acétonitrile, d'acétate d'éthyle ou de méthyl tert-butyl éther et de diméthylsulfoxyde est utilisé, le ratio acétonitrile / diméthylsulfoxyde, acétate d'éthyle / diméthylsulfoxyde ou méthyl tert-butyl éther / diméthylsulfoxyde est préférentiellement compris entre 90/10 m/m et 10/90 m/m, les bornes étant incluses.

La température du milieu lors de la cristallisation ou recristallisation est préférentiellement comprise entre 25°C à 80°C, bornes incluses, plus préférentiellement entre 60 et 80°C, bornes incluses.

Le mélange peut être avantageusement ensemencé pendant l'étape de refroidissement (mode "avec amorce")

Lorsque le mélange n'est pas ensemencé (mode "sans amorce"), le temps de mise en contact avec le mélange de solvants est préférentiellement supérieur à 6h.

L'invention s'étend aussi aux compositions pharmaceutiques renfermant comme principe actif la forme cristalline delta du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La composition pharmaceutique sous la forme de comprimé est de préférence préparée par compression directe.

La posologie utile est adaptable selon la nature et la sévérité de l'affection, la voie d'administration ainsi que l'âge et le poids du patient. Cette posologie varie de 1 à 20 mg par jour en une ou plusieurs prises, préférentiellement de 2,5 à 10 mg en une prise par jour.

Les compositions pharmaceutiques selon l'invention peuvent également contenir un ou plusieurs autres principes actifs choisis parmi les diurétiques tel que l'indapamide, les antagonistes calciques tels que l'amlodipine ou les inhibiteurs du courant If tels que l'ivabradine.

Lorsque les compositions pharmaceutiques selon l'invention contiennent également l'indapamide, la quantité d'indapamide est préférentiellement comprise entre 0,625 et 2,5 mg, les bornes étant incluses.

Lorsque les compositions pharmaceutiques selon l'invention contiennent également l'amlodipine, la quantité d'amlodipine est préférentiellement comprise entre 5 et 10 mg, les bornes étant incluses.

Lorsque les compositions pharmaceutiques selon l'invention contiennent également l'ivabradine, la quantité d'ivabradine est préférentiellement comprise entre 5 et 30 mg, les bornes étant incluses.

Les exemples suivants illustrent l'invention.

Dans les exemples 1 à 4 ci-dessous, le sel d'arginine du périndopril utilisé comme matière première contenait environ 3 à 4% d'eau.
- Figure 1 :: Diffractogramme de la forme delta du périndopril arginine.

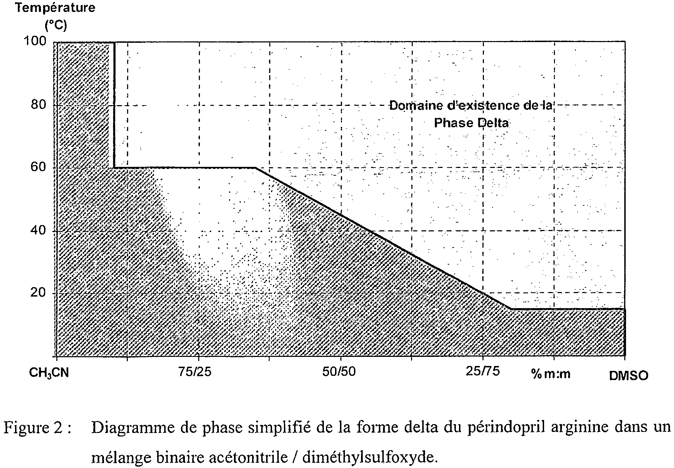

- Figure 2 :: Diagramme de phase de la forme delta du périndopril arginine dans un mélange binaire acétonitrile / diméthylsulfoxyde.
- Figure 3 :: Spectre RMN du solide de la forme delta du périndopril arginine

### Abréviations :

- CPMAS: Cross Polarization Magic Angle Spinning
- DMSO: Diméthylsulfoxyde
- m/m: rapport exprimé en masse/masse
- RMN: Résonance Magnétique Nucléaire

### EXEMPLE 1 : Forme cristalline delta du sel d'arginine du périndopril (mélange binaire acétonitrile / diméthylsulfoxyde 25/75 m/m, mode sans amorce)

Dans un réacteur sont introduits 55,32 g de sel d'arginine du périndopril, 297,50 g de diméthylsulfoxyde et 94,49 g d'acétonitrile.

Le milieu est chauffé sous agitation à 70°C pendant 7 heures, puis refroidi à 40°C en 1°C/min. Après 30 minutes à 40°C, le mélange est filtré sur verre fritté. Le gâteau est lavé avec de l'acétate d'éthyle et séché une nuit à 50°C en étuve ventilée pour conduire à la forme cristalline delta du périndopril arginine avec un rendement de 54%.

### EXEMPLE 2 : Forme cristalline delta du sel d'arginine du périndopril (mélange binaire acétonitrile / diméthylsulfoxyde 25/75 m/m, mode avec amorce)

Dans le réacteur sont introduits 52,2 g de sel d'arginine du périndopril, 216 g de diméthylsulfoxyde et 76 g d'acétonitrile.

Le milieu est chauffé sous agitation à 70°C. A 70°C, 0,52 g de forme delta du périndopril arginine sont additionnés afin d'amorcer la cristallisation.

Le milieu est chauffé à 70°C pendant 5 heures (jusqu'à stabilisation de la courbe de turbidité) puis refroidi à 40°C à 0,5°C/min. Après 30 minutes à 40°C, le mélange est filtré dans une cellule en Inox 1L sur média filtrant (Diamètre = 5 cm, seuil de filtration = 20 microns).

Le gâteau est lavé avec de l'acétate d'éthyle et séché une nuit à 50°C en étuve ventilée.

La forme cristalline delta du périndopril arginine est obtenue avec un rendement de 72% (amorce déduite).

### EXEMPLE 3 : Forme cristalline delta du sel d'arginine du périndopril (mélange binaire acétonitrile / diméthylsulfoxyde 10/90 m/m, mode avec amorce)

Dans le réacteur de 2L sont introduits: 280 g de sel d'arginine du périndopril, 950 g de diméthylsulfoxyde et 97 g d'acétonitrile.

La suspension est chauffée à 80°C. Un passage en solution est observé. Le milieu est maintenu à 80°C pendant 5 minutes puis refroidi à 70°C à la vitesse de 0,5°C/min. Une fois la température du milieu à 70°C, de l'acétonitrile est ajouté (197 g, temps de coulée = 20 minutes). A la fin de l'addition, le milieu reste limpide. La solution est amorcée avec 6 g de forme delta du périndopril arginine. Un palier à 70°C est appliqué pendant 45 minutes.

La suspension est refroidie à 25°C avec une pente de 0,5°C/min. Le temps de contact à 25°C est de 4 heures avant la filtration en cellule 2L. Le gâteau est lavé avec de l'acétate d'éthyle et séché une nuit à 50°C en étuve ventilée. La forme cristalline delta du périndopril arginine est obtenue avec un rendement de 91 % (amorce déduite).

### EXEMPLE 4 : Forme cristalline delta du sel d'arginine du périndopril (mélange binaire acétonitrile / diméthylsulfoxyde 10/90 m/m, mode à 25°C)

Dans un réacteur sous agitation mécanique sont introduits 25 g de sel d'arginine du périndopril et 90 g du binaire acétonitrile/ diméthylsulfoxyde 10/90 (m/m). Après 72 heures de contact à 25°C sous agitation, la transition vers la forme delta est complète.

Le milieu réactionnel est alors filtré pour conduire à l'isolement de la forme cristalline delta du périndopril arginine avec un rendement de 79%.

### EXEMPLE 5 : Forme cristalline delta du sel d'arginine du périndopril à partir du périndopril (acide libre), dans un mélange binaire acétonitrile/DMSO 25/75

Le périndopril (12,5 g, 1 éq.) et la L-arginine (5,32 g - 0,9 éq) sont mis en suspension dans un mélange d'acétonitrile (20 g, d = 0,787) et de DMSO (61 g, d = 1,100). Le milieu réactionnel est chauffé à 50°C pendant une nuit. Le produit est ensuite isolé par filtration sur fritté. Le gâteau est lavé et séché.

La forme cristalline delta du périndopril arginine est obtenue avec un rendement de 79 % par rapport au périndopril.

### EXEMPLE 6 : Forme cristalline delta du sel d'arginine du périndopril (mélange binaire acétate d'éthyle / diméthylsulfoxyde 70/30, mode avec amorce)

Dans un réacteur de 0,5 L sont introduits 15 g de périndopril arginine et 43,6 g de DMSO. La concentration du périndopril arginine dans le milieu est de 25,6 % (pourcentage massique). Le milieu est chauffé à environ 70°C puis 102 g d'acétate d'éthyle sont additionnés en 20 minutes (ratio acétate d'éthyle/DMSO : 70/30 m/m).

Le milieu est amorcé à 70°C avec 0,3 g de forme cristalline delta. Après amorce, le milieu est maintenu sous agitation à 70°C pendant 2 heures. Un refroidissement à 20°C avec une pente de 0,2°C/min est appliqué, suivi d'un temps de contact de 16 heures.

L'isolement du produit a lieu dans une cellule sur média filtrant (Porosité 0,41 µm). Le solide est lavé une fois avec un mélange acétate d'éthyle/DMSO et deux fois avec de l'acétate d'éthyle et séché en étuve sous vide à 50°C.

La forme cristalline delta du périndopril arginine est obtenue avec un rendement de 93 % (amorce déduite).

### EXEMPLE 7 : Composition pharmaceutique

Formule de préparation pour 1000 comprimés dosés à 5 mg :

| | |
|---|---|
| Forme delta du périndopril arginine | 5 g |
| Hydroxypropylcellulose | 2 g |
| Amidon de blé | 10 g |
| Lactose | 100 g |
| Stéarate de magnésium | 3 g |
| Talc | 3 g |

### EXEMPLE 8 : Composition pharmaceutique

Comprimé dosé à 10 mg de périndopril arginine fini à 100 mg :

| | |
|---|---|
| Forme delta du périndopril arginine | 10 mg |
| Lactose monohydrate | 64,2 mg |
| Cellulose microcristalline | 25 mg |
| Stéarate de magnésium | 0,5 mg |
| Silice colloïdale anhydre | 0,3 mg |

### EXEMPLE 9 : Stabilité thermique

La stabilité thermique de la forme delta à 110°C en flacon ouvert a été comparée à celle des formes de l'art antérieur.

Les résultats sont les suivants :

| **Forme cristalline** | **Conditions** | **Pureté HPLC (%)** |
|---|---|---|
| Forme α selon EP 1 989 182 | t=0 | 99,8 |
| | 76 h à 110°C | 98,8 |
| Forme β selon EP 2 016 051 | t=0 | 99,6 |
| | 76 h à 110°C | 98,3 |
| Forme γ selon WO 2009/157018 | t=0 | 99,7 |
| | 76 h à 110°C | 93,4 |
| Forme amorphe | t=0 | 99,3 |
| | 76 h à 110°C | 91,0 |
| Forme obtenue selon le procédé de SI 23001 | t=0 | 99,1 |
| | 76 h à 110°C | 86.9 |
| **Forme δ selon la présente invention** | t=0 | **99,7** |
| | **76 h à 110°C** | **99,5** |

Ces résultats montrent que la forme cristalline delta du sel d'arginine du périndopril a une stabilité thermique améliorée par rapport aux autres formes connues.

## Revendications

1. Forme cristalline delta du sel de L-arginine du périndopril, de formule (I) : 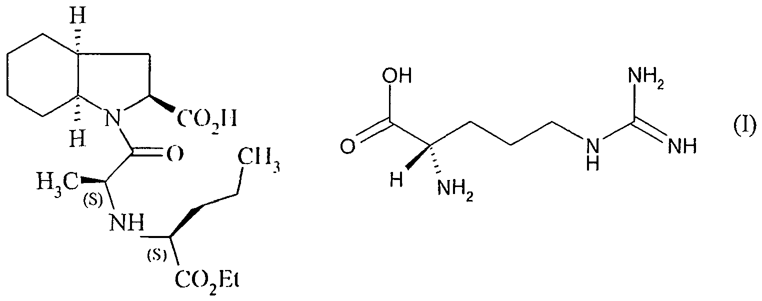 **caractérisée par** les pics de diffraction RX sur poudre suivants, mesurés sur un diffractomètre à anticathode de cuivre et exprimés en termes d'angle de Bragg 2 thêta (°) : 4,3, 11,0, 11,1, 13,2, 14,6, 16,0 et 21,9.

2. Forme cristalline delta du composé de formule (I) selon la revendication 1, **caractérisée par** les pics de diffraction RX sur poudre suivants, mesurés sur un diffractomètre à anticathode de cuivre et exprimés en termes d'angle de Bragg 2 thêta (°) : 4,3, 11,0, 11,1, 11,9, 12,5, 13,2, 14,6, 16,0, 19,2, 19,4, 20,0, 21,9, 22,2 et 22,6.

3. Forme cristalline delta du composé de formule (I) selon la revendication 1, **caractérisée par** le diagramme de diffraction X sur poudre suivant, mesuré sur un diffractomètre à anticathode de cuivre et exprimé en termes de distance inter-réticulaire d, d'angle de Bragg 2 thêta et d'intensité relative, exprimée en pourcentage par rapport à la raie la plus intense :
| **Angle 2 thêta (°)** | **Distance inter-réticulaire d [Å]** | **Intensité relative [%]** |
|---|---|---|
| 4,34 | 20,37 | 66,2 |
| 5,57 | 15,86 | 5,2 |
| 11,04 | 8,02 | 57,5 |
| 11,15 | 7,94 | 47,5 |
| 11,87 | 7,454 | 35,0 |
| 12,47 | 7,09 | 17,9 |
| 13,21 | 6,70 | 33,6 |
| 14,06 | 6,30 | 6,6 |
| 14,64 | 6,05 | 31,8 |
| 16,03 | 5,53 | 17,5 |
| 17,11 | 5,18 | 5,6 |
| 18,27 | 4,85 | 4,1 |
| 19,23 | 4,61 | 100 |
| 19,44 | 4,57 | 17,8 |
| 20,04 | 4,43 | 13,6 |
| 21,11 | 4,21 | 3,7 |
| 21,93 | 4,05 | 23,0 |
| 22,20 | 4,00 | 16,9 |
| 22,61 | 3,93 | 21,2 |
| 23,21 | 3,83 | 4,5 |
| 24,30 | 3,66 | 2,3 |
| 25,09 | 3,55 | 9,4 |
| 25,95 | 3,43 | 1,7 |
| 29,54 | 3,02 | 4,2 |

4. Forme cristalline delta du composé de formule (I) selon la revendication 1, **caractérisée par** un spectre RMN à l'état solide ¹³C CPMAS présentant les pics suivants, exprimés en ppm :
| Pic n° | Déplacement chimique (ppm) | Pic n° | Déplacement chimique (ppm) |
|---|---|---|---|
| 1 | 181,2 | 10 | 38,4 |
| 2 | 180,5 | 11 | 15,6 |
| 3 | 180,1 | 12 | 15,2 |
| 4 | 174,0 | 13 | 15,0 |
| 5 | 173,7 | 14 | 14,5 |
| 6 | 172,7 | | |
| 7 | 172,0 | | |
| 8 | 39,3 | | |
| 9 | 38,8 | | |

5. Procédé de préparation de la forme cristalline delta du composé de formule (I) selon l'une quelconque des revendications 1 à 4, par cristallisation ou recristallisation dans un mélange binaire d'acétonitrile, d'acétate d'éthyle ou de méthyl tert-butyl éther et de diméthylsulfoxyde ou un mélange ternaire d'acétonitrile, de diméthylsulfoxyde et de toluène, à une température supérieure à 20°C.

6. Procédé selon la revendication 5, dans lequel le mélange binaire d'acétonitrile, d'acétate d'éthyle ou de méthyl tert-butyléther et de diméthylsulfoxyde a un ratio acétonitrile / diméthylsulfoxyde, acétate d'éthyle / diméthylsulfoxyde ou méthyl tert-butyléther / diméthylsulfoxyde allant de 90/10 m/m à 10/90 m/m.

7. Procédé selon l'une quelconque des revendications 5 ou 6, dans lequel la température du milieu est comprise entre 25 et 80°C, bornes incluses.

8. Procédé selon la revendication 7, dans lequel le milieu est chauffé à une température de 60 à 80°C.

9. Procédé selon l'une quelconque des revendications 5 à 8, dans lequel le milieu est ensemencé par de la forme cristalline delta.

10. Composition pharmaceutique contenant comme principe actif le composé selon l'une quelconque des revendications 1 à 4, en combinaison avec un ou plusieurs véhicules inertes, non toxiques et pharmaceutiquement acceptables.

11. Composition pharmaceutique selon la revendication 10, **caractérisée en ce qu'**elle contient également un diurétique, un antagoniste calcique ou un inhibiteur du courant If.

12. Composition pharmaceutique selon la revendication 11, **caractérisée en ce que** le diurétique est l'indapamide.

13. Composition pharmaceutique selon la revendication 11, **caractérisée en ce que** l'antagoniste calcique est l'amlodipine.

14. Composition pharmaceutique selon la revendication 11, **caractérisée en ce que** l'inhibiteur du courant If est l'ivabradine.

15. Composé selon l'une quelconque des revendications 1 à 4 pour son utilisation dans le traitement des maladies cardiovasculaires.

16. Composé selon l'une quelconque des revendications 1 à 4 pour son utilisation dans le traitement de l'hypertension artérielle, de l'insuffisance cardiaque ou de la maladie coronaire stable.

## Patentansprüche

1. Delta-Kristallform des L-Argininsalzes von Perindopril der Formel (I): 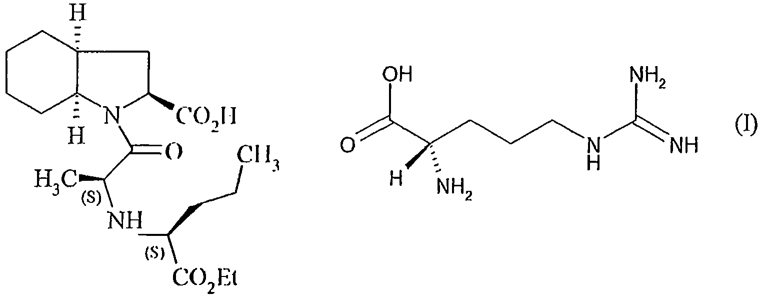 **gekennzeichnet durch** die folgenden mit einem Diffraktometer mit Kupfer-Antikathode gemessenen und als Bragg-2-Theta-Winkel (°) ausgedrückten Pulverröntgenbeugungspeaks: 4,3, 11,0, 11,1, 13,2, 14,6, 16,0 und 21,9.

2. Delta-Kristallform der Verbindung der Formel (I) nach Anspruch 1, **gekennzeichnet durch** die folgenden mit einem Diffraktometer mit Kupfer-Antikathode gemessenen und als Bragg-2-Theta-Winkel (°) ausgedrückten Pulverröntgenbeugungspeaks: 4,3, 11,0, 11,1, 11,9, 12,5, 13,2, 14,6, 16,0, 19,2, 19,4, 20,0, 21,9, 22,2 und 22,6.

3. Delta-Kristallform der Verbindung der Formel (I) nach Anspruch 1, **gekennzeichnet durch** das folgende Pulverröntgenbeugungsdiagramm, gemessen mit einem Diffraktometer mit Kupfer-Antikathode und ausgedückt als Netzebenenabstand d, Bragg-2-Theta-Winkel und relativer Intensität, ausgedrückt als Prozentsatz bezogen auf die intensivste Bande:
| **2-Theta-Winkel (°)** | **Netzebenenabstand d [Å]** | **Relative Intensität [%]** |
|---|---|---|
| 4,34 | 20,37 | 66,2 |
| 5,57 | 15,86 | 5,2 |
| 11,04 | 8,02 | 57.5 |
| 11,15 | 7,94 | 475 |
| 11,87 | 7,454 | 35,0 |
| 12,47 | 7,09 | 17,9 |
| 13,21 | 6,70 | 33,6 |
| 14,06 | 6,30 | 6,6 |
| 14,64 | 6,05 | 31,8 |
| 16,03 | 5,53 | 17,5 |
| 17,11 | 5,18 | 5,6 |
| 18,27 | 4,85 | 4,1 |
| 19,23 | 4,61 | 100 |
| 19,44 | 4,57 | 17,8 |
| 20,04 | 4,43 | 13,6 |
| 21,11 | 4,21 | 3,7 |
| 21,93 | 4,05 | 23,0 |
| 22,20 | 4,00 | 16,9 |
| 22,61 | 3,93 | 21,2 |
| 23,21 | 3,83 | 4,5 |
| 24,30 | 3,66 | 2,3 |
| 25,09 | 3,55 | 9,4 |
| 25,95 | 3,43 | 1,7 |
| 29,54 | 3,02 | 4,2 |

4. Delta-Kristallform der Verbindung der Formel (I) nach Anspruch 1, **gekennzeichnet durch** ein Feststoff⁻¹³C-NMR-Spektrum CPMAS mit den folgenden als ppm ausgedrückten Peaks:
| Peak Nr. | Chemische Verschiebung (ppm) | Peak Nr. | Chemische Verschiebung (ppm) |
|---|---|---|---|
| 1 | 181,2 | 10 | 38,4 |
| 2 | 180,5 | 11 | 15,6 |
| 3 | 180,1 | 12 | 15,2 |
| 4 | 174,0 | 13 | 15,0 |
| 5 | 173,7 | 14 | 14,5 |
| 6 | 172,7 | | |
| 7 | 172,0 | | |
| 8 | 39,3 | | |
| 9 | 38,8 | | |

5. Verfahren zur Herstellung der Delta-Kristallform der Verbindung der Formel (I) nach einem der Ansprüche 1 bis 4 durch Kristallisation oder Umkristallisation in einer binären Mischung aus Acetonitril, Ethylacetat oder Methyl-tert.-butylether und Dimethylsulfoxid oder einer ternären Mischung aus Acetonitril, Dimethylsulfoxid und Toluol bei einer Temperatur von mehr als 20 °C.

6. Verfahren nach Anspruch 5, worin die binäre Mischung aus Acetonitril und Ethylacetat oder Methyl-tert.-butylether und Dimethylsulfoxid ein Acetonitril/Dimethylsulfoxid- bzw. Ethylacetat/Dimethylsulfoxid- oder Methyl-tert.-butylether/Di-methylsulfoxid-Verhältnis von 90/10 m/m bis 10/90 m/m aufweist.

7. Verfahren nach einem der Ansprüche 5 oder 6, worin die Temperatur des Mediums zwischen 25 und 80 °C einschließlich der Grenzen liegt.

8. Verfahren nach Anspruch 7, worin die Mischung auf eine Temperatur von 60 bis 80 °C erhitzt wird.

9. Verfahren nach einem der Ansprüche 5 bis 8, worin das Medium mit der Delta-Kristallform angeimpft wird.

10. Pharmazeutische Zubereitung enthaltend als Wirkstoff die Verbindung nach einem der Ansprüche 1 bis 4 in Kombination mit einem oder mehreren inerten, nichttoxischen und pharmazeutisch annehmbaren Hilfsstoffen.

11. Pharmazeutische Zubereitung nach Anspruch 10, **dadurch gekennzeichnet, dass** sie gleichzeitig ein Diuretikum, einen Calciumantagonisten oder einen If-Strominhibitor enthält.

12. Pharmazeutische Zubereitung nach Anspruch 11, **dadurch gekennzeichnet, dass** das Diuretikum Indapamid ist.

13. Pharmazeutische Zubereitung nach Anspruch 11, **dadurch gekennzeichnet, dass** der Calciumantagonist Amlodipin ist.

14. Pharmazeutische Zubereitung nach Anspruch 11, **dadurch gekennzeichnet, dass** der If-Strominhibitor Ivabradin ist.

15. Verbindung nach einem der Ansprüche 1 bis 4 zur Verwendung bei der Behandlung von kardiovaskulären Erkrankungen.

16. Verbindung nach einem der Ansprüche 1 bis 4 zur Verwendung bei der Behandlung von arterieller Hypertension, Herzinsuffizienz oder stabiler Koronarerkrankung.

## Claims

1. Delta crystalline form of perindopril L-arginine salt of formula (I): 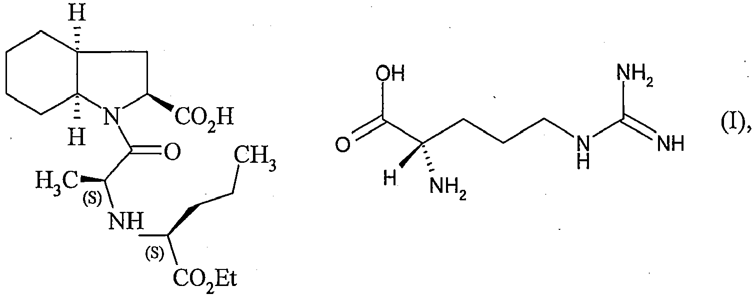 **characterised by** the following X-ray powder diffraction peaks measured using a diffractometer with a copper anticathode and expressed in terms of Bragg's angle 2 theta (°): 4.3, 11.0, 11.1, 13.2, 14.6, 16.0 and 21.9.

2. Delta crystalline form of the compound of formula (I) according to claim 1, **characterised by** the following X-ray powder diffraction peaks measured using a diffractometer with a copper anticathode and expressed in terms of Bragg's angle 2 theta (°): 4.3, 11.0, 11.1, 11.9, 12.5, 13.2, 14.6, 16.0, 19.2, 19.4, 20.0, 21.9, 22.2 and 22.6.

3. Delta crystalline form of the compound of formula (I) according to claim 1, **characterised by** the following X-ray powder diffraction diagram measured using a diffractometer with a copper anticathode and expressed in terms of interplanar spacing d, Bragg's angle 2 theta, and relative intensity expressed as a percentage in relation to the most intense line:
| **Angle 2 theta (°)** | **Interplanar distance d [Å]** | **Relative intensity [%]** |
|---|---|---|
| 4.34 | 20.37 | 66.2 |
| 5.57 | 15.86 | 5.2 |
| 11.04 | 8.02 | 57.5 |
| 11.15 | 7.94 | 47.5 |
| 11.87 | 7.454 | 35.0 |
| 12.47 | 7.09 | 17.9 |
| 13.21 | 6.70 | 33.6 |
| 14.06 | 6.30 | 6.6 |
| 14.64 | 6.05 | 31.8 |
| 16.03 | 5.53 | 17.5 |
| 17.11 | 5.18 | 5.6 |
| 18.27 | 4.85 | 4.1 |
| 19.23 | 4.61 | 100 |
| 19.44 | 4.57 | 17.8 |
| 20.04 | 4.43 | 13.6 |
| 21.11 | 4.21 | 3.7 |
| 21.93 | 4.05 | 23.0 |
| 22.20 | 4.00 | 16.9 |
| 22.61 | 3.93 | 21.2 |
| 23.21 | 3.83 | 4.5 |
| 24.30 | 3.66 | 2.3 |
| 25.09 | 3.55 | 9.4 |
| 25.95 | 3.43 | 1.7 |
| 29.54 | 3.02 | 4.2 |

4. Delta crystalline form of the compound of formula (I) according to claim 1, **characterised by** a solid-state ¹³C CPMAS NMR spectrum having the following peaks, expressed in ppm:
| Peak no. | Chemical shift (ppm) | Peak no. | Chemical shift (ppm) |
|---|---|---|---|
| 1 | 181.2 | 10 | 38.4 |
| 2 | 180.5 | 11 | 15.6 |
| 3 | 180.1 | 12 | 15.2 |
| 4 | 174.0 | 13 | 15.0 |
| 5 | 173.7 | 14 | 14.5 |
| 6 | 172.7 | | |
| 7 | 172.0 | | |
| 8 | 39.3 | | |
| 9 | 38.8 | | |

5. Process for the preparation of the delta crystallisation form of the compound of formula (I) according to any one of claims 1 to 4, by crystallisation or recrystallisation from a binary mixture of acetonitrile, ethyl acetate or methyl tert-butyl ether and dimethyl sulphoxide or a ternary mixture of acetonitrile, dimethyl sulphoxide and toluene, at a temperature higher than 20°C.

6. Process according to claim 5, wherein the binary mixture of acetonitrile, ethyl acetate or methyl tert-butyl ether and dimethyl sulphoxide has a ratio of acetonitrile/dimethyl sulphoxide, ethyl acetate/dimethyl sulphoxide or methyl tert-butyl ether/dimethyl sulphoxide ranging from 90/10 w/w to 10/90 w/w.

7. Process according to either claim 5 or claim 6, wherein the temperature of the medium is between 25 and 80°C, inclusive.

8. Process according to claim 7, wherein the mixture is heated to a temperature of from 60 to 80°C.

9. Process according to any one of claims 5 to 8, wherein the mixture is seeded with the delta crystalline form.

10. Pharmaceutical composition comprising, as active ingredient, the compound according to any one of claims 1 to 4, in combination with one or more inert, non-toxic, pharmaceutically acceptable carriers.

11. Pharmaceutical composition according to claim 10, **characterised in that** it also comprises a diuretic, a calcium antagonist or an If current inhibitor.

12. Pharmaceutical composition according to claim 11, **characterised in that** the diuretic is indapamide.

13. Pharmaceutical composition according to claim 11, **characterised in that** the calcium antagonist is amlodipine.

14. Pharmaceutical composition according to claim 11, **characterised in that** the If current inhibitor is ivabradine.

15. Compound according to any one of claims 1 to 4 for use in the treatment of cardiovascular diseases.

16. Compound according to any one of claims 1 to 4 for use in the treatment of arterial hypertension, heart failure or stable coronary disease.
